# EUROPEAN PATENT APPLICATION

(11) **EP 2 052 717 A1**
(43) Date of publication of application: **29.04.2009**
(21) Application number: 09002123.9
(22) Date of filing: 25.01.2005
(51) Int. Cl.: A61K 9/16, A61K 9/22, A61K 9/52, A61K 31/135, A61K 31/137, A61P 25/24, A61P 1/00

(54) **Multiparticulate O-desmethylvenlafaxine salts and uses thereof**

(30) Priority: 06.02.2004 US 542384 P
(62) Divisional of application: 05711931.5
(71) Applicant: Wyeth, Madison, NJ 07940 (US)
(72) Inventor: Diorio, Christopher Richard, Campbell Hall, New York 10916 (US); Shah, Syed M., East Hanover, New Jersey 07936 (US); Fawzi, Mahdi B., Morristown, New Jersey 07960 (US)
(74) Representative: Fletcher, Rodney Justin

(57) **Abstract**

A multiparticulate O-desmethylvenlafaxine (ODV) succinate or formate is described. Methods of treating depression and reducing the gastrointestinal side-effects of ODV are also described.

## Description

### BACKGROUND OF THE APPLICATION

O-desmethylvenlafaxine (ODV), the major metabolite of venlafaxine, selectively blocks the reuptake of serotonin and norepinephrine. Klamerus, K. J. et al., "Introduction of the Composite Parameter to the Pharmacokinetics of Venlafaxine and its Active O-Desmethyl Metabolite", J. Clin. Pharmacol. 32:716-724 (1992). O-desmethyl-venlafaxine, chemically named 1-[2-(dimethylamino)-1-(4-phenol)ethyl]-cyclohexanol, was exemplified as a fumarate salt in U.S. Pat. No. 4,535,186. However, the fumarate salt of O-desmethyl-ventafaxine has unsuitable physicochemical and permeability characteristics. O-desmethyl-venlafaxine is also exemplified as a free base in International Patent Publication No. WO 00/32555.

The succinate form of ODV has been described [US Patent 6,673,838]. The succinate monohydrate form of ODV has been incorporated into an extended release hydro-gel tablet, which reduces adverse effects such as nausea, vomiting, diarrhea, and abdominal pain. Formulations describing the use of hydroxypropyl methylcellulose (HPMC) as the hydrogel matrix have been described [WO 02/064543 A2].

However, the effects of the hydrogel formulation have been observed to be variable when the ODV hydrogel tablet is given with food.

### SUMMARY OF THE INVENTION

The present invention provides a multiparticulate form of ODV that reduces undesirable characteristics associated with ODV and the hydrogel formulation thereof. These ODV multiparticulates are composed of ODV succinate, ODV formate, or combinations thereof

Advantageously, this formulation also allows more convenient dosing to patients who have difficulty swallowing solid foods.

### DETAILED DESCRIPTION OF THE INVENTION

The present invention provides a multiparticulate formulation of an O-desmethylvenlafaxine (ODV) that contains a multiparticulate form of ODV Succinate (DVS), ODV formate (DVF), or a combination thereof.

The use of a multiparticulate fonnulation facilitates dosing to pediatric patients, geriatric patients, and patients who have trouble swallowing, by dispersing the spheroids in a suspending liquid or sprinkling /dispersing in a low pH liquid like applesauce, prior to administration. The smaller size of the multiparticulates, in a capsule or pouch or any other container, also allows dosing through nasogastric or gastrostomy tube.

Suitably, the multiparticulate ODV is a spheroid, bead or pellet, which is generally in the range of about 0.6 mm to about 1 mm in size. However, this may vary in size, without departing from the present invention.

The multiparticulate ODV of the invention are composed, at a minimum, of a core composed of DVS, DVF or a combination thereof, and one or more diluents, binders, fillers, glidants, anti-adherents, a pH adjuster and/or an adjuvant.

DVS is prepared as described in US Patent 6,673,838, which is incorporated by reference herein. The formate salt of ODV (DVF), described in published US Patent Application Publn No. US 2003/0236309, which is incorporated by reference herein, can be prepared using similar techniques by substitution of the appropriate salt. The multiparticulate core contains about 3% w/w to about 70% w/w DVS and/or DVF. In other embodiments, the DVS or DVF can range from about 5% w/w to about 60% w/w, from about 10 % w/w to about 50% w/w, from about 20 % w/w to about 40% w/w, or from about 25% w/w to about 35% w/w, about 30 % w/w to about 45% w/w, or about 32% to about 44% w/w, based upon 100% weight of the uncoated dosage form.

Suitably, the total amount of diluent, binders, fillers, glidants, anti-adherents, and adjuvants present in the core is an amount of about 30% w/w to about 97% w/w of the multiparticulate core. For example, when present, a binder, diluent and/or filler can each be present in an amount of about 15 % w/w to about 80 % w/w, or about 20% w/w to about 70 % w/w, or about 25% w/w to about 45% w/w, or about 30% w/w to about 42 % w/w of the uncoated dosage form. The total amount of a pH adjuster in the formulation can range from about 0.1% w/w to about 10% w/w of the core, or about 1% w/w to about 8% w/w, or about 3% w/w to about 7% w/w. However, these percentages can be adjusted as needed or desired by one of skill in the art.

The binder may be selected from among known binders, including, e.g., cellulose, and povidone, among others. In one embodiment, the binder is selected from among microcrystalline cellulose, crospovidone, and mixtures thereof.

Suitable pH adjusters include, e.g., sodium carbonate, sodium bicarbonate, potassium carbonate, lithium carbonate, among others. Still other suitable components will be readily apparent to one of skill in the art.

In one embodiment, the DVS and/or DVF is in a sustained release formulation which contains rate-controlling components. Typically, such rate controlling components are rate controlling polymers selected from among hydrophilic polymers and inert plasticized polymers. Suitable rate controlling hydrophilic polymers include, without limitation, polyvinyl alcohol (PVA), hypomellose and mixtures thereof. Examples of suitable insoluble or inert "plastic" polymers include, without limitation, one or more polymethacrylates (i.e., Eudragit® polymer). Other suitable rate-controlling polymer materials include, e.g., hydroxyalkyl celluloses, poly(ethylene) oxides, alkyl celluloses, carboxymethyl celluloses, hydrophilic cellulose derivatives, and polyethylene glycol.

In one embodiment, an ODV multiparticulate of the invention contains about 5% w/w to about 75% w/w microcrystalline cellulose (MCC), about 10 % w/w to about 70% w/w MCC, about 20% w/w to about 60 % w/w, or about 30 % w/w to about 50% w/w, based on the weight of the uncoated dosage unit.

In one embodiment, the multiparticulate DVS or DVF-containing core is uncoated. The multiparticulates can be placed into a suitable capsule shell or compressed into tablets, using techniques know to those of skill in the art. Suitably, the results capsule shell or compressed tablets contain 10 mg to 400 mg of ODV.

In other embodiments, the multiparticulate ODV contain one or more coatings over the core. In still other embodiments, the multiparticulate consists of a pellet core and non-functional seal coating and a functional second coating.

In one embodiment, an initial seal coat can be applied directly to the core. Although the components of this seal coat can be modified by one of skill in the art, the seal coat may be selected from among suitable polymers such as hydroxypropyl methylcellulose (HPMC), ethylcellulose, polyvinyl alcohol, and combinations thereof, optionally containing plasticizers and other desirable components. A particularly suitable seal coat contains HPMC. For example, a suitable seal coat can be applied as a HPMC solution at a concentration of about 3% w/w to 25% w/w, and preferably 5% w/w to about 7.5% w/w. Upon drying, under suitable conditions, the initial seal coat is in the range of about 1% w/w to about 3% w/w, or about 2% w/w, of the uncoated multiparticulate. In another embodiment, a commercially available seal coat containing HPMC, among other inert components, is utilized. One such commercially available seal coat is Opadry® Clear (Colorcon, Inc.).

Optionally, the multiparticulates can contain a further coating layer over the initial seal coat, if present, or directly to the uncoated multiparticulate ODV core, to provide a delay release formulation. These formulations may also lower the incidence of the side effects, including nausea, emesis, and irritable bowel syndrome. Without wishing to be bound by theory, it is believed that these side-effects are avoided by bypassing release in the upper GI tract and providing release in the lower GI tract.

An enteric coat (rate-controlling film) may be applied to the multiparticulates and may include, but is not limited to polymethacrylates, hypomellose, and ethylcellulose, or a combination thereof. The modified release multiparticulate formulation can contain from about 3 % w/w to about 70% w/w of DVS, DVF, or a combination thereof, and from about 5% w/w to about 75% w/w microcrystalline cellulose, based on the weight of an uncoated dosage form.

In one embodiment, the enteric coat contains a product which is a copolymer of methacrylic acid and methacrylates, such as the commercially available Eudragit® L 30 K55 (Röhm GmbH & Co. KG). Suitably, this enteric coat is applied such that it coats the multiparticulate in an amount of about 15 to 45 % w/w, or about 20 % w/w to about 30% w/w, or about 25% w/w to 30% w/w of the uncoated or initially-coated multiparticulate. In one embodiment, the enteric coat is composed of a Eudragit® L30D-55 copolymer (Röhm GmbH & Co. KG), talc, triethyl citrate, and water. More particularly, the enteric coating may contain about 30% w/w of a 30 wt% dispersion of Eudragit® L 30 D55 coating; about 15% w/w talc, about 3% triethyl citrate; a pH adjuster such as sodium hydroxide and water.

In another embodiment, the enteric coat contains an ethylcellulose-based product, such as the commercially available Surelease® aqueous ethylcellulose dispersion (25% solids) product (Colorcon, Inc.). In one embodiment, a solution of Surelease® dispersion of about 3% w/w to about 25% w/w, and preferably about 3% to about 7%, or about 5% w/w, is applied to the multiparticulate. Upon drying under suitable conditions, the enteric coat is in the range of about 2% to about 5%, or about 3% to about 4% w/w of the uncoated or initially-coated multiparticulate.

In one embodiment, the enteric-coated multiparticulate is further coated with a final seal coat. Suitably, this final seal coat is composed of HPMC and water, upon drying, is less than about 1 wt% of the total, coated multiparticulate.

### II. Method of Producing Multiparticulate Formulations of Invention

The.multiparticulate formulations of the invention can be prepared using the techniques described herein, as well as methods known to those of skill in the art.

In one embodiment, the uncoated ODV multiparticulates are prepared as follows. The dry components, including, at least the DVS and/or DVF and the binder are dry blended in a suitable mixer, e.g., a Hobart mixer. Optionally, the HPMC and a pH adjuster may be included in this step. Subsequently, the liquid components, e.g., the surfactant and water, are mixed in to afford a granulated product. The granulation is then extruded and spheronized through a suitable device (e.g., a Nica extruder/spheronizer) and the resulting spheroids are dried, sifted, and optionally blender prior to storage.

In another embodiment, the uncoated ODV multiparticulates are prepared as follows. The DVS and binder are combined and granulated with water in a planetary mixer, such as a Hobart mixer. Then using the Nica® System the resulting wet mass is extruded through a 1 mm screen. The extrudates are then transferred to a spheronizer and spun until spherical pellets are obtained (approximately 2 to 3 minutes). The wet pellets are then dried in an Aeromatic Strea^{™} fluid bed dryer to a moisture level of 2% to 5%. The dried pellets are then passed through a mesh screen to remove larger oversize pellets.

Optionally, an initial seal coat can be applied to the uncoated multiparticulates. For example, an initial seal coat can be applied on a fluid bed coater, e.g., by spraying. In one embodiment, an Aeromatic Strea^{™} fluid bed apparatus is fitted with a Wurster column and bottom spray nozzle system. Approximately 200 grams of the dried pellet cores are charged into the unit. The Opadry® Clear seal coat is applied with an inlet temperature of approximately 50°C to 60°C, a coating solution spray rate of 5 to 10 grams/minute, atomization pressure of 1 to 2 bar. The desired product temperature is 35°C to 45°C, and preferably 38°C to 43°C.

The enteric coat can be applied directly to the uncoated spheroid core, i.e., the uncoated multiparticulate, or may be applied over an initial seal coat. The enteric coat, as described above, is typically applied on a fluid bed coater. In one embodiment, Surelease® aqueous ethylcellulose dispersion (25% solids) is applied in a similar fashion as the seal coat. After the ethylcellulose coat is applied, the pellets are dried for an additional 5 to 10 minutes. They are then removed and screened through a mesh screen to remove agglomerates and oversize particles.

In one embodiment, a final seal coat is applied over the enteric coat and, optionally, talc is utilized as a final step prior to filling the multiparticulates into a suitable packaging unit.

### III. Formulations/Kits/Methods of delivery

In another embodiment, the present invention provides products containing the ODV multiparticulates of the invention.

In one embodiment, the ODV muitiparticulates are packaged for use by the patient or his caregiver. For example, the multiparticulates can be packaged in a foil or other suitable package and is suitable for mixing into a food product (e.g., applesauce or the like) or into a drink for consumption by the patient.

In another embodiment, the ODV multiparticulates are suspended in a physiologically compatible suspending liquid. For oral liquid pharmaceutical compositions, pharmaceutical carriers and excipients can include, but are not limited to water, glycols, Oils, alcohols, flavoring agents, preservatives, coloring agents, and the like.

In yet another embodiment, the ODV multiparticulates are filled in capsules, caplets or the like for oral delivery.

In another embodiment, the present invention provides for the use of multiparticulate formulations of the invention in the preparation of medicaments, including but not limited to medicaments useful in the treatment of depression, gastrointestinal side-effects of venlafaxine in a subject undergoing treatment therewith, and irritable bowel syndrome.

In another embodiment, the present invention provides for the use of multiparticulate formulations of the invention in the preparation of medicaments for delivery to a pediatric or geriatric patient.

In other embodiments, the present invention provides for the use of multiparticulate formulations of the invention in the preparation of dosing units, including but not limited to dosing units for oral, transdermal, or mucosal administration.

Also encompassed by the invention are pharmaceutical packs and kits comprising a container, such as a foil package or other suitable container, having a formulation of the invention in unit dosage form.

In still a further embodiment, the invention provides method of treating a subject in need thereof by administering an effective dose of the ODV multiparticulates of the invention. The formulations of the invention are useful in treatment of depression, anxiety, panic disorder, generalized anxiety disorder, post traumatic stress disorder, premenstrual dysphoric disorder, fibromyalgia, agorophobia, attention deficit disorder, obsessive compulsory disorder, social anxiety disorder, autism, schizophrenia, obesity, anorexia nervosa, bulimia nervosa, Gilles de la Tourette Syndrome, vasomotor flushing, cocaine and alcohol addiction, sexual dysfunction, borderline personality disorder, chronic fatigue syndrome, urinary incontinence, pain, Shy Drager syndrome, Raynaud's syndrome, Parkinson's disease, and epilepsy. These formulations are also useful for enhancing cognition or treating cognitive impairment in a patient, cessation of smoking or other tobacco uses in a patient, treating hypothalamic amenorrhea in a depressed or non-depressed human female, lowering the incidence of nausea, vomiting, diarrhea, abdominal pain, headache, vaso-vagal malaise, or trismus resulting from the oral administration of O-desmethylvenlafaxine succinate.

Suitably, the multiparticulate ODV of the invention can reduce the gastrointestinal side-effects of venlafaxine in a subject undergoing treatment therewith comprising administering to the patent a formulation of the invention.

An effective amount of the multiparticulate of the invention is an amount sufficient to prevent, inhibit, or alleviate one or more symptoms of the aforementioned conditions. The dosage amount useful to treat, prevent, inhibit or alleviate each of the aforementioned conditions will vary with the severity of the condition to be treated and the route of administration. The dose, and dose frequency will also vary according to age, body weight, response and past medical history of the individual human patient. In generally the recommended daily dose range for the conditions described herein lie within the range of 10 mg to about 1000 mg DVS or DVF per day and more preferably within the range of about 15 mg to about 350 mg/day and still more preferably from about 15 mg to about 140 mg/day. In other embodiments of the invention the dosage will range from about 30 mg to about 90 mg/day. Dosage is described in terms of the free base and is adjusted accordingly for the succinate salt. In managing the patient, is generally preferred that the therapy be initiated at a lower dose and increased if necessary. Dosages for non-human patients can be adjusted accordingly by one skilled in the art.

A DVS or DVF multiparticulate may also be provided in combination with other active agents including, e.g., venlafaxine. The dosage of venlafaxine is preferably about 75 mg to about 350 mg/day and more preferably about 75 mg to about 225 mg/day. Still more preferably the dosage of venlafaxine is about 75 mg to about 150 mg/day. The ratio of DVS and/or DVF multiparticulate will vary from patient to patient depending upon a patient's response rate, but generally will be at least 6:1 ODV salt to venlafaxine. Venlafaxine or another active agent delivered in a regimen with the multiparticulate of the invention may be formulated together with the multiparticulate of the invention, or delivered separately.

Any suitable route of administration can be employed for providing the patient with an effective amount of ODV multiparticulate. For example, oral, mucosal (e.g. nasal, sublingual, buccal, rectal or vaginal), parental (e.g. intravenous or intramuscular), transdermal, and subcutaneous routes can be employed. Preferred routes of administration include oral, transdermal and mucosal.

Multiparticulate DVS and/or DVF can be combined with a pharmaceutical carrier or excipient (e.g., pharmaceutically acceptable carriers and excipients) according to conventional pharmaceutical compounding technique to form a pharmaceutical composition or dosage form. Suitable pharmaceutically acceptable carriers and excipients include, but are not limited to, those described in Remington's, The Science and Practice of Pharmacy, (Gennaro, A. R., ed., 19th edition, 1995, Mack Pub. Co.) which is herein incorporated by reference. The phrase "pharmaceutically acceptable" refers to additives or compositions that are physiologically tolerable and do not typically produce an allergic or similar untoward reaction, such as gastric upset, dizziness and the like, when administered to an animal, such as a mammal (e.g., a human).

Oral solid pharmaceutical compositions may include, but are not limited to starches, sugars, microcrystalline cellulose, diluents, granulating agents, lubricants, binders and disintegrating agents. The pharmaceutical composition and dosage form may also include venlafaxine or a salt thereof as discussed above.

The following examples illustrate exemplary dosage forms of the multiparticulate ODV of the invention, and the use thereof. These examples are not a limitation on the present invention.

### EXAMPLE 1- Multiparticulate ODV Succinate (DVS) Formulations

### Multiparticulate Dosage Form A

| Ingredient | mg/300 mg | %w/w |
|---|---|---|
| DVS | 151.75 (100 as ODV free base) | 50.58 |
| Microcrystalline cellulose | 148.25 | 49.42 |
| Total | 300 | 100.00 |

### Multiparticulate Dosage Form B

| Ingredient | mg/300 mg | %w/w |
|---|---|---|
| DVS | 151.75 (100 as ODV free base) | 50.58 |
| Hypomellose | 75.0 | 25.00 |
| Microcrystalline cellulose | 73.25 | 24.42 |
| Total | 300 | 100.00 |

### EXAMPLE 2 - Multiparticulate ODV Formate (DVF) Formulations

### Dosage Form A for DVF

| Ingredient | mg/300 mg | %w/w |
|---|---|---|
| DVF 118.3 | (100 as ODV free base) | 39.43 |
| Microcrystalline cellulose | 181.7 | 60.57 |
| Total | 300 | 100.00 |

### Multiparticulate Dosage Form B for DVF

| Ingredient | mg/300 mg | %w/w |
|---|---|---|
| DVF | 118.3 (100 as ODV free base) | 39.43 |
| Hypomellose | 90.85 | 30.28 |
| Microcrystalline cellulose | 90.85 | 30.28 |
| Total | 300 | 100.00 |

The multiparticulate formulation is anticipated to provide a sustained, therapeutically effective plasma level over at least a 16 to 20 hour period. Similar to the hypomellose tablet model [See, US Patent 6,673,838 and US 6,274,171], the time to peak plasma levels (Tmax) is expected to be generally three to ten hours, more preferably 6 to 9 hours. The Tmax is critical for the reduction in adverse effects such as nausea and vomiting by bypassing the receptors in the upper GI tract, which are responsible for these adverse effects. Moreover, the increases in the Cmax (peak concentration) and AUC (total area under the concentration-time curve) observed with the hypomellose table in the fed state will not be observed in the multiparticulate formulation due to quicker elimination from the stomach accompanied by a smaller quantity of dissolved drug passing from the stomach to the upper GI tract. The multiparticulate formulation will also demonstrate a reduction of patient-to-patient variability as well as dose to dose variability due to the fact that they can easily pass through the pylorus whereas a hypomellose table may have difficulty, especially after some tablet swelling has occurred. The modified release pellet formula also demonstrates a reduction of nausea, vomiting, diarrhea, abdominal pain, headache, vaso-vaginal malaise, and/or trisumus.

### EXAMPLE 3 - Preparation of Coated Desvenlafaxine succinate (DVS) Multiparticulates Formulation

| **Ingredient** | **Grams/2000 grams** | **% wt/wt** |
|---|---|---|
| DVS | 1400.0 | 70.0 |
| Microcrystalline cellulose | 600.0 | 30.0 |
| Water | Qs | qs |

The manufacturing of the multiparticulate core was as follows. The desvenlafaxine succinate (DVS) is combined with microcrystalline cellulose and granulated with water in a planetary mixer. Then using the Nica® system the resulting wet mass is extruded through a 1 mm screen. The DVS extrudates are then transferred to the spheronizer and spun at approximately 700 rpm until spherical pellets are obtained (2-3 minutes).

The wet pellets are then dried in an Aeromatic Strea^{™} fluid bed dryer to a moisture level of 2% to 5%. The dried pellets are passed through an 18 mesh screen to remove larger oversize pellets. The pellets are now ready for the coating process.

### Coating

### Seal Coat

| **Ingredient** | **Grams/500 grams** | **% wt/wt** |
|---|---|---|
| Opadry® Clear (HPMC) | 25.0 | 5 |
| Water | 475.0 | 95.0 |

The Aeromatic Strea^{™} fluid bed apparatus is fitted with a Wurster column and bottom spray nozzle system. Approximately 200 grams of the dried pellet cores are charged into the unit. The Opadry® seal coat is applied with an inlet temperature of approximately 60°C, a coating solution spray rate of 5-10 grams/minute, atomization pressure of I-2 bar. The desired product temperature is 38°C-43°C. After approximately a 2% weight gain of the seal coat is achieved the ethylcellulose coat can be applied.

### Ethylcellulose coat

| **Ingredient** | **Grams/500 grams** | **% wt/wt** |
|---|---|---|
| Surelease® (aqueous ethylcellulose dispersion 25% solids) | 25.0 | 5 |
| Water | 475.0 | 95.0 |

The ethylcellulose is applied in a similar fashion as the seal coat to a weight gain of 3-4%. After the ethylcellulose coat is applied, the pellets are dried for an additional 5-10 minutes. They are removed and screened through an 18 mesh screen to remove agglomerates and oversize particles.

### Dissolution Testing of Coated DVS Multiparticulates

The resulting coated multiparticulates were tested in-vitro for the DVS-233 release rate. Pellets containing DVS multiparticulates having an active amount equivalent to 150 mg of desvenlafaxine were tested using USP Apparatus I, baskets at 100 rpm. The dissolution media was 0.9% NaCl at 37°C.

The results demonstrate that the uncoated DVS multiparticulate product provided immediate release (< 1 hour), which was sustained for at least 24 hours. The coated DVS multiparticulate product had delayed release [< 50% release after 2 hours, < 70% release after 4 hours, and < 90% release after 8 hours] with maximum sustained release levels achieved after 20 hours.

### EXAMPLE 4 - Multiparticulate ODV Succinate (DVS) Formulation

| **Ingredient** | mg/tablet |
|---|---|
| DVS-233 | 303.5 |
| Microcrystalline cellulose | 231.35 |
| povidone | 25 |

| **Controlled Release Coat** | |
|---|---|
| Ethylcellulose dispersion, NF (15% solids) | 85 |
| Water* | NA |

| **Enteric Coat** | |
|---|---|
| Eudragit L30D-55 Dispersion (30% solids) | 56 |
| Triethyl citrate | 6 |
| Sodium hydroxide | 9 |
| talc | 30 |
| Water* | NA |

| | |
|---|---|
| * Does not appear in final product | |

The present invention is not to be limited in scope by the specific embodiments described herein. Various modifications to these embodiments will be obvious to one of skill in the art from the description. Such modifications fall within the scope of the appended claims.

Patents, patent applications, publications, procedures and the like are cited thoughout the application. The disclosures of these documents are incorporated by reference herein in their entireties. To the extent that a conflict may exist between the specification and a reference, the language of the disclosure made herein controls.

## Claims

1. A multiparticulate formulation of an O-desmethylvenlafaxine (ODV) comprising a multiparticulate form of ODV succinate and/or ODV formate.

2. The formulation according to claim 1, wherein said multiparticulate formulation is a sustained release formulation or a delayed release formulation.

3. The formulation according to claim 1 or claim 2, wherein said formulation comprises spheroids, beads or pellets of an ODV.

4. The formulation according to claim 3, wherein the pellets are about 0.6 mm to about 1 mm in size.

5. The formulation according to any one of claims 1 to 4, wherein the multiparticulate further comprises rate controlling polymers.

6. The formulation according to claim 5, wherein the rate controlling polymers are selected from the group consisting of hydrophilic polymers and inert plasticized polymers.

7. The formulation according to claim 1, wherein the multiparticles further comprise a diluent, filler, glidant, anti adherent, and/or an adjutant.

8. The formulation according to any one of claims 1 to 7, wherein the multiparticles comprise a film coating.

9. A method for treating depression in a subject in need thereof, comprising administering to the patient a composition comprising a multiparticulate formulation of an ODV succinate or formate according to any of claims 1-8.

10. A method for reducing the gastrointestinal side-effects of venlafaxine in a subject undergoing treatment therewith comprising administering to the patent a composition comprising a multiparticulate formulation of an ODV according to any of claims 1-8.

11. A modified release formulation of a multiparticulate comprising about 3 % w/w to about 70% w/w O-desmethylvenlafaxine succinate or formate and about 5% w/w to about 75% w/w microcrystalline cellulose.

12. The modified release formulation according to claim 11, wherein the multiparticulate further comprise a seal coat.

13. The modified release formulation according to claim 12, wherein the seal coat comprises hydroxypropyl methylcellulose.

14. The modified release formulation according to any of claims 11-13, wherein the multiparticulate further comprises an enteric coat selected from among polymethylacrylates, hypomellose, ethylcellulose and combinations thereof.

15. The modified release formulation according to claim 14, wherein the enteric coat comprises ethylcellulose.

16. A method for treating depression in a subject in need thereof, comprising administering to the patient a modified formulation according to any one of claims 11-15.

17. A method for reducing the gastrointestinal side-effects of an ODV in a subject undergoing treatment therewith comprising administering to the patent a modified formulation according to any of claims 11-15.

18. A modified release venlafaxine product comprising 10 mg to 400 mg of multiparticulate O-desmethylvenlafaxine (ODV) succinate or formate.

19. The product according to claim 18 that is a capsule shell containing said multiparticulate ODV succinate or format.

20. The product according to claim 18 that is a compressed tablet comprising said multiparticulate ODV succinate or formate.

21. A method for treating depression in a subject in need thereof, comprising administering to the patient a regimen comprising a product according to claim 18.

22. A method for reducing the gastrointestinal side-effects associated with venlafaxine treatment, said method comprising administering to the patent a regimen comprising a product according to any one of claims 18 to 20.

23. A method for delivering ODV to a pediatric or geriatric patient, said method comprising the step of administering pellets, beads or spheroids according to claim 3 or claim 4.

24. A product comprising multiparticulate ODV succinate and/or formate.

25. The product according to claim 24 which comprises a foil packet comprising the multiparticulate ODV succinate and/or formate.

26. A multiparticulate formulation of ODV formate or succinate that can be administered for patients through naso-gastric tubes, as a suspension or dispersed over semi-solid foods.

27. A delayed release multiparticulate formulation of ODV formate or succinate for treatment of irritable bowel syndrome.

28. Use of a formulation according to any of claims 1-8, 11-15, and 26-27 in preparing a medicament.

29. Use according to claim 28, wherein said medicament is useful in the treatment of depression, gastrointestinal side-effects of venlafaxine in a subject undergoing treatment therewith, and irritable bowel syndrome.

30. Use of a formulation according to claims 3 or 4 in the preparation of a medicament for delivery to a pediatric or geriatric patient.

31. Use of a formulation according to any of claims 1-8, 11-15, and 26-27 in preparing a dosing unit.

32. Use according to claim 31, wherein said dosing unit is for oral, transdermal, or mucosal administration.

33. A pharmaceutical pack comprising a container having a formulation according to any of claims 1-8, 11-15, and 26-27 in unit dosage form.
